(19) Europäisches Patentamt
European Patent Office
Office européen des brevets

(11) Veröffentlichungsnummer : **0 336 250 B1**

(12) # EUROPÄISCHE PATENTSCHRIFT

(45) Veröffentlichungstag der Patentschrift : **09.09.92 Patentblatt 92/37**

(51) Int. Cl.$^5$ : **C07D 233/90,** C07D 207/40

(21) Anmeldenummer : **89105365.4**

(22) Anmeldetag : **25.03.89**

(54) **Verfahren zur Herstellung von 5-gliedrigen, stickstoffhaltigen Heteroaromaten.**

Verbunden mit 89904057.0/0362350 (europäische Anmeldenummer/Veröffentlichungsnummer) durch Entscheidung vom 06.08.91.

(30) Priorität : **07.04.88 DE 3811621**

(43) Veröffentlichungstag der Anmeldung : **11.10.89 Patentblatt 89/41**

(45) Bekanntmachung des Hinweises auf die Patenterteilung : **09.09.92 Patentblatt 92/37**

(84) Benannte Vertragsstaaten : **AT BE CH DE ES FR GB IT LI NL SE**

(56) Entgegenhaltungen : **EP-A- 0 207 563**

(56) Entgegenhaltungen :
**EP-A- 0 306 868**
**FR-A- 1 184 709**
**JP-A-61 178 968**
**JP-A-62 175 471**

(73) Patentinhaber : **MERCK PATENT GESELLSCHAFT MIT BESCHRÄNKTER HAFTUNG**
**Frankfurter Strasse 250 Postfach 4119**
**W-6100 Darmstadt (DE)**

(72) Erfinder : **Kirchlechner, Richard, Dr.**
**Leitenweg 7 a**
**W-8093 Rott (DE)**
Erfinder : **Casutt, Michael, Dr.**
**Am Ohlenberg 10**
**W-6106 Erzhausen (DE)**
Erfinder : **Basedow, Arno, Prof. Dr.**
**Elisabethenstrasse 25**
**W-6368 Bad Vilbel (DE)**

EP 0 336 250 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Verbindungen Formel I

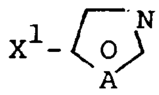

I

worin

A NR[1] oder CH-X[2],

X[1] und X[2] jeweils unabhängig voneinander CO-OR[2], CO-NR[3]R[4] oder CN,

R[1] und R[2] jeweils unabhängig voneinander Wasserstoff, Alkyl mit 1-8 C-Atomen, oder einen carbocyclischen Rest und

R[3] und R[4] jeweils unabhängig voneinander Alkyl mit 1-7 C-Atomen, Aryl mit 6-8 C-Atomen oder Aralkyl mit 7-13 C-Atomen oder auch jeweils zusammen mit dem benachbarten Stickstoffatom einen heterocyclischen Rest mit 2-6 C-Atomen, wobei auch eine $CH_2$-Gruppe durch O, S oder NH ersetzt sein kann, bedeuten.

Der Erfindung lag die Aufgabe zugrunde, ein neues Verfahren für die Herstellung der Verbindungen der Formel I bereitzustellen, das diese Verbindungen mit Hilfe einfacher Reaktionen in hoher Ausbeute zugänglich macht.

Verbindungen der Formel I sind wertvolle Synthesezwischenprodukte; insbesondere werden die Verbindungen der Formel Ia,

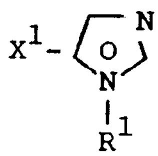

Ia

worin R[1] und X[1] die angegebene Bedeutung besitzen, bei der Synthese von Imidazol-Alkaloiden wie z.B. Isomacrorin oder Pilocarpin eingesetzt.

Ia ($X^1$ = $COOR^2$) kann nach EP-OS 0207563 ausgehend von N-Alkylglycinester-Hydrochloriden in 4 Stufen hergestellt werden.

Aus dem N-Methylacetaminomalonsäurediethylester wird Ia ($R^1 = CH_3$, $X^1 = COOR^2$) in einer 5stufigen Synthese erhalten.

In einem weiteren 5stufigen Verfahren kann Diaminomaleonitril mit Orthoameisensäuretriethylester zum Imidazol-4,5-dinitril umgesetzt werden, welches nach Alkylierung mit Dimethylsulfat, Hydrolyse und partieller Decarboxylierung durch Erhitzen in Acetanhydrid die Verbindung der Formel Ia ($R^1 = CH_3$, $X^1 = COOH$) ergibt.

Alle diese Verfahren sind jedoch durch eine hohe Zahl von Syntheseschritten und damit eine niedrige Gesamtausbeute gekennzeichnet.

Obwohl bekannt ist, daß man bei Umsetzung von 3-Dimethylamino-2-azaprop-2-en-1-yliden-dimethylammoniumchlorid (Gold's Reagenz) mit Hydrazinen 1,3,4-Triazole erhält, gibt es keinen Hinweis, daß sich dieses Reagenz auch zur Herstellung von anderen 5gliedrigen Heterocyclen verwenden läßt.

Es wurde nun überraschend gefunden, daß Verbindungen der Formel I, insbesondere der Formel Ia, durch Umsetzung von Methylen-Verbindungen der Formel II oder deren Säureadditionssalzen mit Aminomethylenformamidinium-Salzen der Formel III in einem einzigen Syntheseschritt, im Eintopfverfahren und in hohen Ausbeuten hergestellt werden können.

Gegenstand der Erfindung ist daher ein Verfahren zur Herstellung von Verbindungen der Formel I

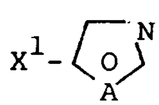

I

worin

A NR[1] oder CH-$X^2$,

$X^1$ und $X^2$ jeweils unabhängig voneinander CO-$OR^2$, CO-$NR^3R^4$ oder CN, und

$R^1$ und $R^2$ jeweils unabhängig voneinander Wasserstoff, Alkyl mit 1-8 C-Atomen oder einen carbocyclischen Rest, und

$R^3$ und $R^4$ jeweils unabhängig voneinander Alkyl mit 1-7 C-Atomen, Aryl mit 6-8 C-Atomen oder Aralkyl mit 7-13 C-Atomen oder auch jeweils zusammen mit dem benachbarten Stickstoffatom einen heterocyclischen Rest mit 2-6 C-Atomen, wobei auch eine $CH_2$-Gruppe durch O, S oder NH ersetzt sein kann,

bedeuten, dadurch gekennzeichnet, daß man eine Methylen-Verbindung der Formel II

$$X^1\text{-}CH_2\text{-}AH \qquad II$$

oder eines ihrer Säureadditionssalze, worin $X^1$ und A die angegebene Bedeutung besitzen, mit einem Salz der Formel III,

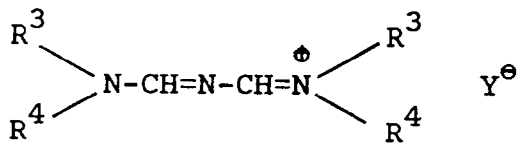

III

worin

$R^3$ und $R^4$ die angegebene Bedeutung besitzen, und

$Y^\ominus$ $Cl^\ominus$, $Br^\ominus$, $J^\ominus$, $J_3^\ominus$, $ClO_4^\ominus$ oder $BF_4^\ominus$ bedeutet,

und einer Base umsetzt.

Gegenstand der Erfindung ist insbesondere ein Verfahren zur Herstellung der Verbindungen der Formel Ia, worin A $NR^1$ bedeutet,

und die Verbindung der Formel IIa in Form eines Säureadditionssalzes mit einer Verbindung der Formel III umgesetzt wird.

Gegenstand der Erfindung ist ferner ein verfahren zur Herstellung von Pilocarpin, indem man zunächst eine Verbindung der Formel I nach mindestens einem Verfahren, wie inden Patentansprüchen 1-5 wiedergegeben, herstellt und sie dann in an sich bekanntes Weise weiter zu Pilocarpin umsetzt.

Das erfindungsgemäße Verfahren ergibt unabhängig von der Beschaffenheit der Gruppen A, $X^1$, $X^2$, $R^1$, $R^2$, $R^3$, $R^4$ und $X^\ominus$ die entsprechenden Verbindungen der Formel I in durchweg hohen Ausbeuten.

Falls A $NR^1$ und $X^1$ $COOR^2$ bedeutet, sind $R^1$ und $R^2$ jeweils unabhängig voneinander Wasserstoff, Alkyl mit 1-7-C-Atomen oder carbocyclische Reste.

Falls $R^1$ und/oder $R^2$ einen Alkylrest bedeuten, so kann dieser geradkettig oder verzweigt sein. Dieser bedeutet somit Methyl, Ethyl, n-Propyl, n-Butyl, n-Pentyl, n-Hexyl, n-Heptyl, n-Octyl, i-Propyl, 1-(bzw. 2-)Methylpropyl, tert.-Butyl, 1-(2- bzw. 3-)Methylbutyl, neo-Pentyl, 1-(2-, 3-bzw. 4-)Methylpentyl, 1- (2-, 3-, 4- bzw. 5-)Methylhexyl oder 2-Ethylhexyl (i-Octyl). Vorzugsweise bedeutet er Methyl, Ethyl oder i-Propyl, insbesondere Methyl.

Falls $R^1$ und/oder $R^2$ einen carbocyclischen Rest bedeuten, so kann dieser aromatisch, cycloaliphatisch oder araliphatisch sein. Dieser bedeutet somit bevorzugt Phenyl, Benzyl, Cyclohexyl, 1-Indanyl, Tetrahydronaphthyl (z.B. 1,2,3,4-Tetrahydro-1-naphthyl), Benzocycloheptyl, (z.B. 5-Benzocycloheptyl), 9,10-Dihydro-9-anthracenyl, 9H-Fluoren-9-yl, 5-Dibenzo[a,d]cycloheptyl oder Dihydronaphthyl (z.B. 1,2-Dihydro-1-naphthyl).

Die vorstehend genannten carbocyclischen Reste können jeweils unsubstituiert oder mit 1 bis 6 Substituenten, ausgewählt aus der Gruppe Alkyl, Alkoxy mit 1-5 C-Atomen und Halogen, substituiert sein.

Die Gruppe $R^3R^4N$- bedeutet vorzugsweise einen N,N-Dimethyl-, N,N-Diethyl-, N,N-Dipropyl-, N,N-Diisopropyl-, N,N-Dibutyl-, N,N-Diisobutyl-, N,N-Di-sek-butyl-, N,N-Dicyclohexyl-, N,N-Dibenzyl-, N,N-Diphenyl- oder N,N-Di-(o-, m- oder p-Tolyl)-amino-Rest bzw. einen Morpholino-, Piperidino- oder N-Methylanilinorest.

Für den Reaktionsablauf ebenfalls unkritisch ist die Bedeutung des Anions $Y^\ominus$; es bedeutet bevorzugt $Cl^\ominus$ oder $Br^\ominus$.

Die Ausgangsstoffe der Formel II sind bekannt (IIa: A = $NR^1$: Glycin- bzw. Aminoacetonitril-Derivate; IIb: A = CH-$X^2$: Bernsteinsäure-Derivate) oder können nach bekannten Methoden, wie sie in der Literatur beschrieben (z.B. in Standardwerken wie Houben-Weyl, Methoden der Organischen Chemie, Georg-Thieme-Verlag, Stuttgart) sind, und zwar nach Reaktionsbedingungen, wie sie für die genannten Umsetzungen bekannt und geeignet sind, hergestellt werden. Dabei kann man auch von an sich bekannten, hier nicht näher erwähnten Varianten Gebrauch machen.

Die Ausgangsstoffe der Formel III sind ebenfalls bekannt und können aus Cyanurchlorid und N,N-Dialkylformamiden hergestellt werden.

Die Umsetzung der Methylen-Verbindung der Formel II mit dem Salz der Formel III erfolgt vorzugsweise in einem inerten Lösungsmittel in Anwesenheit einer Base. Als Basen eignen sich in Abhängigkeit von der C-H-Acidität der eingesetzten Methylen-Verbindung z.B. Alkali- bzw. Erdalkalimetallhydroxide wie Lithium-, Calcium-, Barium-, Natrium- oder Kaliumhydroxid, Alkalimetallcarbonate wie Natrium- oder Kaliumcarbonat, Alkoholate wie Natriummethylat, Natriumethylat, Lithiumethylat oder Kalium-tert.-butylat, Alkalimetallamide wie Kalium- oder Natriumamid, oder organische Basen wie Triethylamin, Pyridin, 4-N,N-Dimethylaminopyridin, Lutidin, Piperidin, Morpholin, Piperazin, Collidin oder Chinolin, Lithiumdiisopropylamid oder Lithiumtetramethylpiperidid.

Zweckmäßig führt man die Reaktion in einem inerten Lösungsmittel durch. Als inerte Lösungsmittel eignen sich vorzugsweise Ether wie Diethylether, Ethylenglykoldimethylether, Tetrahydrofuran, tert.-Butylmethylether oder Dioxan sowie Amide wie Dimethylformamid, N,N-Dimethylpropylenharnstoff, Dimethylacetamid oder N-Methylpyrrolidon, ferner Sulfoxide wie Dimethylsulfoxid oder Sulfone wie Sulfolan sowie Kohlenwasserstoffe wie Pentan, Hexan, Cyclohexan, Benzol oder Toluol. Die Reaktionstemperaturen liegen zweckmäßig entsprechend der Reaktivität der eingesetzten Methylen-Verbindung zwischen -78 °C und +150 °C, vorzugsweise zwischen +20 °C und +100 °C, die Reaktionszeiten zwischen 1 und 48 Stunden.

Bei der Umsetzung der Verbindungen der Formel II, worin $X^1$ CO-$OR^2$ bedeutet, mit dem Salz der Formel III können, bedingt durch die Reaktivität dieser Edukte, sowohl die Carbonsäureester der Formel I, worin $X^1$ CO-$OR^2$ bedeutet, als auch die Carbonsäureamide der Formel I, worin $X^1$ CO-$NR^3R^4$ bedeutet, entstehen. Das Verhältnis der entstehenden Produkte läßt sich durch geeignete Wahl der Reaktionsbedingungen leicht steuern.

Zur vollständigen Unterdrückung der Bildung von Carbonsäureamiden der Formel I ist von Vorteil, die Reaktion in Gegenwart von nicht enolisierbaren Carbonsäurestern durch zuführen, die geeignet sind, die bei der Reaktion aus dem Salz der Formel III gebildeten Amine abzufangen. Besonders geeignete nicht enolisierbare Carbonsäureester sind die Methyl- oder Ethylester der entsprechenden Carbonsäure wie z.B. Benzoesäure-, Phtalsäure- oder Terephtalsäure, der Perfluoralkylsäuren, wie z.B. Trifluoressigsäure, oder der aliphatischen Carbonsäure, welche keine zur Carboxylgruppe $\alpha$-ständige Wasserstoffatome aufweist, wie z.B. Pivalylsäure oder Oxalsäure.

Andererseits lassen sich die Carbonsäureamide der Formel I, worin $X^1$ bzw. $X^1$ und $X^2$ CO-$NR^3R^4$ bedeutet, auch gezielt herstellen, indem man die Reaktion ohne Zusatz solcher Carbonsäureestern bei erhöhter Temperatur und längerer Reaktionszeit durchführt.

Bevorzugt werden Verbindungen der Formel IIa (A bedeutet $NR^1$) in Form ihrer Säure-Additionssalze IIa′

$$X^1\text{-}CH_2\text{-}NHR^1 \,.\, HY^1 \qquad IIa'$$

eingesetzt.

Als $Y^1$ kommen anorganische Säurereste wie F, Cl, Br, J, $J_3$, $HSO_4$, $H_2PO_4$ oder $ClO_4$, aber auch organische Säurereste wie Carboxylate, vor allem Acetat oder Trifluoracetat, oder Sulfonate, vor allem p-Toluolsulfonat, Trifluormethansulfonat oder Methansulfonat, in Betracht. Insbesondere bevorzugt sind die Chloride.

In einer besonders bevorzugten Ausführungsform des erfindungsgemäßen Verfahrens wird ein Hydrochlorid der Formel IIa ($Y^1$ = Cl) zusammen mit einem Aminomethylenformamidiniumchlorid (Y = Cl) der Formel III bei -10 °C bis +30 °C zu einem Gemisch eines Alkalimetallalkoholates, vorzugsweise Natriummethylat oder Natriumethylat, und eines inerten Lösungsmittels, vorzugsweise eines Kohlenwasserstoffes wie Cyclohexan oder Toluol oder eines Ethers, wie z.B. Dioxan oder Tetrahydrofuran, gegeben und anschließend 1 bis 30 Stunden bei Temperaturen zwischen 0° und 130 °C gerührt.

Verbindungen der Formel I, insbesondere der Formel Ia, sind bekannt und können nach bekannten Methoden, wie z.B. in Helv. Chim. Acta 55, 1053-1062 (1972) oder J. Org. Chem 51, 1713-1719 (1986) beschrieben, zu Pilocarpin umgesetzt werden.

Die nach dem erfindungsgemäßen Verfahren herstellbaren 5-gliedrigen, stickstoffhaltigen Heterocyclen sind darüberhinaus wertvolle Ausgangsstoffe für die Herstellung von Farbstoffen, Pflanzenschutzmitteln und weiteren Pharmazeutika oder eignen sich selbst, wie z.B. in EP-OS 0207563 offenbart, als Mittel zum Beeinflussen des Pflanzenwachstums.

Das erfindungsgemäße Verfahren erlaubt somit die Herstellung von Verbindungen der Formel I, insbesondere der Formel Ia, auf einfache Weise in hohen Ausbeuten aus leicht zugänglichen, wohlfeilen Ausgangsmaterialien in einem einzigen, im Eintopfverfahren durchzuführenden Syntheseschritt und stellt damit einen wesentlichen Fortschritt auf dem Gebiet der Synthese von Verbindungen der Formel I, insbesondere bei der Pilocarpin-Synthese, dar.

Die folgenden Beispiele sollen die Erfindung erläutern, ohne sie zu begrenzen:

Beispiel 1

In eine unter Stickstoff gehaltene Suspension von 16,21 g Natriummethylat in 300 ml Toluol trägt man bei Raumtemperatur nacheinander 17,45 g Sarcosinmethylester-hydrochlorid sowie 31,09 g 3-Dimethylamino-2-azaprop-2-en-1-yliden-dimethylammoniumchlorid ein und rührt das Gemisch 24 Stunden bei 70 °C. Danach dekantiert man ab, extrahiert den Rückstand dreimal mit Toluol und engt ein.

Nach Chromatographie des Rückstandes an Kieselgel (Ethylacetat) werden 15,1 g 1-Methyl-1H-imidazol-5-carbonsäuremethylester erhalten; Kp: 115 °C/8 torr, Sublimation bei 25 °C/0,01 torr, F. 56 °C.

Analog erhält man mit Bernsteinsäuredimethylester den Pyrrol-3,4-dicarbonsäure-dimethylester;

mit N-(9-Fluorenyl)-glycin-methylester-hydrochlorid den 1-(9-Fluorenyl)-1H-imidazol-5-carbonsäuremethylester;

mit N-(1,2,3,4-Tetrahydronaphthalen-1-yl)-glycinmethylester-hydrochlorid den 1-(1,2,3,4-Tetrahydronaphthalen-1-yl)-1-H-imidazol-5-carbonsäuremethylester, F. 63 °C;

mit N-Methylaminoacetonitril das 1-Methyl-1H-imidazol-5-carbonitril;

mit N-Benzyl-glycin-benzylester-hydrochlorid den 1-Benzyl-1H-imidazol-5-carbonsäurebenzylester;

mit Natriumethylat/Sarcosinethylester-hydrochlorid erhält man 1-Methyl-1H-imidazol-5-carbonsäureethylester;

mit N-(9-Fluorenyl)-glycinbenzylester-hydrochlorid erhält man 1-(9-Fluorenyl)-1H-imidazol-5-carbonsäurebenzylester.

Mit Glycinester-hydrochloriden erhält man analog:

1-H-Imidazol-5-carbonsäuremethylester
1-H-Imidazol-5-carbonsäureethylester, F. 158 °C
1-H-Imidazol-5-carbonsäurebenzylester

Mit N-substituierten Glycinester-hydrochloriden erhält man analog:

1-Isopropyl-1-H-imidazol-5-carbonsäuremethylester, Kp 130 °C/13 Torr
1-Cyclohexyl-1-H-imidazol-5-carbonsäuremethylester, F 90 °C
1-Benzyl-1-H-imidazol-5-carbonsäuremethylester, F 64 °C
1-Phenyl-1-H-imidazol-5-carbonsäureethylester, F 81 °C
1-(2-Ethylhexyl)-1H-imidazol-5-carbonsäuremethylester

Beispiel 2

Man legt unter Stickstoff bei Raumtemperatur eine Suspension von 189,07 g Natriummethylat in 2000 ml Dioxan vor, trägt unter Rühren nacheinander 181,45 g Sarcosinmethylester-Hydrochlorid und 261,84 g 3-Dimethylamino-2-azaprop-2-en-1-yliden-dimethylammoniumchlorid ein und rührt das Reaktionsgemisch 24 Stunden bei 60 °C.

Danach wird abgesaugt, mit Dioxan nachgewaschen und das Filtrat eingeengt.

Nach Chromatographie des Rückstandes an Kieselgel (Ethylacetat) werden 152,1 g 1-Methyl-1H-imidazol-5-carbonsäuremethylester, F. 55 °C bis 56 °C erhalten.

$^1H$-NMR (200 MHz, $CDCl_3$): $\delta$ = 7,72 (brs, 1H); 7,55 (brs, 1H); 3,90 (s, 3H); 3,84 (s, 3H).

Beispiel 3

Man legt unter Stickstoff bei Raumtemperatur eine Susspension von 54,03 g Natriummethylat in 600 ml Dioxan vor, trägt nacheinander 41,87 g Sarcosinmethylester-Hydrochlorid, 65,46 g 3-Dimethylamino-2-azaprop-2-en-1-yliden-dimethylammoniumchlorid sowie 47,24 g Oxalsäuredimethylester ein und rührt das Reaktionsgemisch 12 Stunden bei 65 °C. Danach wird über Kieselgur abgesaugt, mit Dioxan nachgewaschen und unter vermindertem Druck eingeengt.

Nach Chromatographie des Rückstandes an Kieselgel (Ethylacetat) werden 33,2 g 1-Methyl-1H-imidazol-5-carbonsäuremethylester mit einem Schmelzpunkt von 55 °C erhalten.

Beispiel 4

In eine unter Stickstoff gehaltene Suspension von 16,21 g Natriummethylat in 200 ml Tetrahydrofuran trägt man bei Raumtemperatur nacheinander 13,96 g Sarcosinmethylester-Hydrochlorid und 21,27 g 3-Dimethylamino-2-azaprop-2-en-1-yliden-dimethylammoniumchlorid ein und rührt das Gemisch 6 Stunden bei Rückflußtemperatur.

Danach kühlt man auf Raumtemperatur ab, filtriert über Kieselgur, wäscht mit Tetrahydrofuran nach und

engt unter vermindertem Druck ein.

Nach Chromatographie des Rückstandes an Kieselgel (Ethylacetat) werden 11,3 g 1-Methyl-1H-imidazol-5-carbonsäuremethylester erhalten; Fp. 55-56 °C.

Beispiel 5

Man legt unter Stickstoff bei Raumtemperatur eine Suspension von 162 g Natriummethylat in 2000 ml Dioxan vor, trägt unter Rühren nacheinander 139,6 g Sarcosinmethylester-Hydrochlorid und 212,7 g 3-Dimethylamino-2-azaprop-2-en-1-yliden-dimethylammoniumchlorid ein und rührt das Reaktionsgemisch 24 Stunden bei 90 °C.

Danach wird abgesaugt, mit Dioxan nachgewaschen und das Filtrat unter vermindertem Druck eingeengt.

Nach Destillation des Rückstandes im Vakuum werden 89,3 g 1-Methyl-1H-imidazol-5-carbonsäuredimethylamid erhalten; F. 46-47 °C.

Beispiel 6

Zu einem unter Stickstoff gehaltenen Gemisch aus Lithiumdiisopropylamid (hergestellt aus 3,0 g Diisopropylamin und 19 ml einer 15%igen Lösung von n-Butyllithium in Hexan) und 15 ml Tetrahydrofuran gibt man bei -78 °C ein Gemisch von 4,5 g Bernsteinsäuredibenzylester und 10 ml Tetrahydrofuran. Nach Aufwärmen auf 0 °C gibt man 3,1 g 3-Dimethylamino-2-azaprop-2-en-1-yliden-dimethylammoniumchlorid hinzu und rührt das Gemisch 24 Stunden bei Siedetemperatur. Nach Zugabe von 25 ml einer gesättigten Ammoniumchlorid-Lösung extrahiert man die wässrige Phase dreimal mit Ether und engt ein. Nach Chromatographie des Rückstandes an Kieselgel (Ethylacetat) erhält man Pyrrol-3,4-dicarbonsäurebenzylester.

Analog werden dargestellt

Pyrrol-3,4-dicarbonsäuremethylester
Pyrrol-3,4-dicarbonsäureethylester
Pyrrol-3,4-dicarbonsäureisopropylester
4-Cyanopyrrol-3-carbonsäuremethylester
3,4-Dicyanopyrrol

Anwendungsbeispiel A

Kalium-hydrid (4,15 g, 35%ige Öl-Suspension) wird 2mal mit 15 ml Hexan gewaschen und in 150 ml Diethylenglykol suspendiert. Zu diesem Gemisch gibt man bei 0 °C 11,6 g Diethyl(cyan(1-(tert-butoxycarbonyl)-propyl)-methyl)-phosphonat (hergestellt nach Lit.[1]). Nach 1stündigem Rühren bei Raumtemperatur gibt man ein Gemisch aus 4,0 g 1-Methyl-1-H-imidazol-5-carboxaldehyd (hergestellt aus 1-Methyl-1-H-imidazolcarbonsäuremethylester nach Lit.[2]) und 20 ml Diethylenglykol hinzu und rührt 12 Stunden bei Raumtemperatur. Nach Zugabe von Wasser und Trennen der Phasen wird die wässrige Phase 3mal mit 200 ml Ether extrahiert. Nach Einengen erhält man ein E/Z-Gemisch von 3-Cyan-2-ethyl-4-(1-methyl-1H-5-imidazolyl)-3-butensäure-tert.-butylester. Aus diesem erhält man nach Lit.[1] in 5 Stufen (+)-Isopilocarpin (F. 159 °C, $\alpha_D$ = +34,3° (c = 1,804, Wasser) nach Überführung in das Nitrat), aus dem (+) Pilocarpin durch Epimerisierung erhalten wird:

Zu einer Lösung von 0,21 ml Diisopropylamin in Tetrahydrofuran gibt man bei 0 °C 0,97 ml einer 15%igen Lösung von n-Butyllithium in Hexan. Nach 15minütigem Rühren und Abkühlen auf -78 °C fügt man 100 mg (+)-Isopilocarpin gelöst in 1 ml Tetrahydrofuran hinzu und rührt 10 Stunden bei -78 °C. Nach Zugabe von 1 g 2,6-Di-tert-butyl-4-methylphenol, Aufwärmen auf Raumtemperatur und Hinzufügen von 15 ml Salzsäure (0,5 n) werden die Phasen getrennt. Die wässrige Phase wird 2mal mit 25 ml Chloroform gewaschen. Nach Einengen der organischen Phase und präparativer Trennung an einer HPLC-Säule erhält man optisch reines (+)-Pilocarpin, das mit Salpetersäure (65%ig) in Ethanol in das Pilocarpinnitrat, F. 174 °C, $\alpha_D$ = +81° (c = 1,618, Wasser), übergeführt wird.

Lit.[1]: R.S. Compagnone, H. Rapoport, J, Org. Chem. 51, 1713-1719 ( 1986)
Lit.[2]: H. Link, K. Bernauer, Helv. Chim. Acta 55, 1053-1062 (1972)

**Patentansprüche**

1. Verfahren zur Herstellung von Verbindungen der Formel I

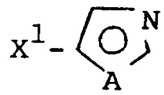

I

worin

A NR¹ oder CH-X²,

X¹ und X² jeweils unabhängig voneinander CO-OR², CO-NR³R⁴ oder CN,

R¹ und R² jeweils unabhängig voneinander Wasserstoff, Alkyl mit 1-8 C-Atomen oder einen carbocyclischen Rest, und

R³ und R⁴ jeweils unabhängig voneinander Alkyl mit 1-7 C-Atomen, Aryl mit 6-8 C-Atomen oder Aralkyl mit 7-13 C-Atomen oder auch jeweils zusammen mit dem benachbarten Stickstoffatom einen heterocyclischen Rest mit 2-6 C-Atomen, wobei auch eine $CH_2$-Gruppe durch O, S oder NH ersetzt sein kann,

bedeuten, dadurch gekennzeichnet, daß man eine Methylen-Verbindung der Formel II

$X^1$-$CH_2$-AH II

oder deren Säure-Additions-Salz, worin X¹ und A die angegebene Bedeutung besitzen, mit einem N,N'-tetrasubstituierten Aminomethylenformamidinium-Salz der Formel III

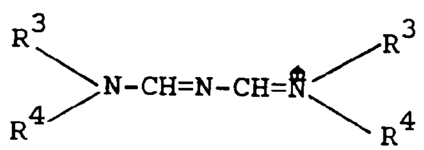

$Y^{\ominus}$ III

worin

R³ und R⁴ die angegebene Bedeutung besitzen, und

$Y^{\ominus}$ $Cl^{\ominus}$, $Br^{\ominus}$, $J^{\ominus}$, $J_3^{\ominus}$, $ClO_4^{\ominus}$ oder $BF_4^{\ominus}$ bedeutet,

und einer Base umsetzt.

2. Verfahren nach Anspruch 1, dadurch gekennzeichnet, daß A NR¹ bedeutet und daß die Verbindung der Formel II in Form eines Säure-Additions-Salzes mit einer Verbindung der Formel III umgesetzt wird.

3. Verfahren nach Anspruch 1 oder 2, dadurch gekennzeichnet, daß man als Base Metallalkoholate verwendet.

4. Verfahren zur Herstellung der Verbindungen der Formel I, worin X¹ CO-OR² bedeutet, nach Anspruch 2 oder 3, dadurch gekennzeichnet, daß man die Reaktion in Gegenwart eines nicht-enolisierbaren Carbonsäureesters durchführt.

5. Verfahren nach Anspruch 4, dadurch gekennzeichnet, daß man als nicht-enolisierbaren Carbonsäureester Oxalsäuredimethylester einsetzt.

6. Verfahren zur Herstellung von Pilocarpin, dadurch gekennzeichnet, daß man eine Verbindung der Formel I nach mindestens einem der Ansprüche 1-5 herstellt und sie in an sich bekannter Weise weiter zu Pilocarpin umsetzt.

## Claims

1. Process for the preparation of compounds of the formula I

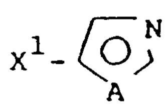

I

in which
A is NR or CH-$X^2$,
$X^1$ and $X^2$ are each independently of one another CO-$OR^2$, CO-$NR^3R^4$ or CN,
$R^1$ and $R^2$ are each independently of one another hydrogen, alkyl having 1-8 C atoms or a carbocyclic radical and
$R^3$ and $R^4$ are each independently of one another alkyl having 1-7 C atoms, aryl having 6-8 C atoms or aralkyl having 7-13 C atoms or are each together with the adjacent nitrogen atom also a heterocyclic radical having 2-6 C atoms, in wich a $CH_2$ group can also be replaced by O, S or NH,
characterized in that a methylene compound of the formula II

$X^1$-$CH_2$-AH II

or one of its acid addition salts in which $X^1$ and A have the meaning mentioned is reacted with an N,N-tetrasubstituted aminomethyleneformamidinium salt of of the formula III

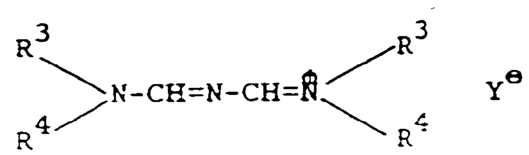

III

in which
R3 and R4 have the meaning mentioned and
$Y^{\ominus}$ is $Cl^{\ominus}$, $Br^{\ominus}$, $I^{\ominus}$, $I_3^{\ominus}$, $ClO_4^{\ominus}$ or $BF_4^{\ominus}$,
and a base.

2. Process according to Claim 1, characterized in that A is $NR^1$ and the compound of the formula II is reacted in the form of an acid addition salt with a compound of the formula III.

3. Process according to Claim 1 or 2, characterized in that the bases used are metal alcoholates.

4. Process for the preparation of compounds of the formula I in which $X^1$ is CO-$OR^2$ according to Claim 2 or 3, characterized in that the reaction is carried out in the presence of an non-enolizable carboxylic ester.

5. Process according to Claim 4, characterized in that the non-enolizable carboxylic ester is dimethyl oxalate.

6. Process for the preparation of pilocarpine, characterized in that a compound of the formula I is prepared according to at least one of Claims 1-5 and reacted in a manner known per se to give pilocarpine.

**Revendications**

1 - Procédé pour la préparation des composés de formule I

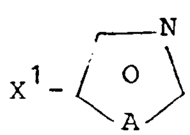

I

où
A représente $NR^1$ ou CH-$X^2$,
$X^1$ et $X^2$ représentent, indépendammment l'un de l'autre, CO-$OR^2$, CO-$NR^3R^4$ ou CN,
$R^1$ et $R^2$ représentent, indépendamment l'un de l'autre, l'hydrogène, un alkyle ayant 1 à 8 atomes de C ou un reste carbocyclique et
$R^3$ et $R^4$ représentent, indépendamment l'un de l'autre, un alkyle ayant 1 à 7 atomes de C, un aryle ayant 6 à 8 atomes de C ou un aralkyle avant 7 à 13 atomes de C ou représentent également ensemble avec l'atome d'azote voisin un reste hétérocyclique ayant 2 à 6 atomes de C, un groupe $CH_2$ pouvant être remplacé par O, S ou NH,
caractérisé en ce que l'on fait réagir un composé méthylénique de formule II

$X^1$-$CH_2$-AH II

ou l'un de ses sels d'addition d'acide où $X^1$ et A ont la signification précédemment indiquée, avec un sel d'aminométhylèneformamidinium N,N'-tétrasubstitué de formule III

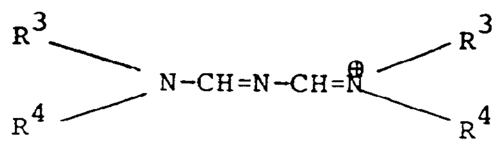

Y$^{\ominus}$ III

où

$R^3$ et $R^4$ ont la signification précédemment indiquée et

$Y^{\ominus}$ représente $Cl^{\ominus}$, $Br^{\ominus}$, $I^{\ominus}$, $I_3^{\ominus}$, $ClO_4^{\ominus}$, ou $BF_4^{\ominus}$ et une base.

2 - Procédé selon la revendication 1, caractérisé en ce que A représente $NR^1$ et en ce que le composé ce formule II sous forme d'un sel d'addition d'acide est mis à réagir avec un composé de formule III.

3 - Procédé selon la revendication 1 ou 2, caractérisé en ce que l'on utilise comme base des alccolates métalliques.

4 - Procédé pour la préparation des composés de formule I où $X^1$ représente CO-$OR^2$, selon la revendication 2 ou 3, caractérisé en ce que l'on effectue la réaction en présence d'un ester d'acide carboxylique non énolisable.

5 - Procédé selon la revendication 4, caractérisé en ce que l'on utilise l'ester diméthylique de l'acide oxalique comme ester d'acide carboxylique non énolisable.

6 - Procédé pour la préparation de la pilocarpine, caractérisé en ce que l'on prépare un composé de formule I selon au moins l'une des revendications 1 à 5 et en ce qu'on le transforme ensuite en pilocarpine d'une façon connue en soi.